Europäisches Patentamt

European Patent Office

Office européen des brevets

(11) EP 1 310 288 A1

(19)

(12) **EUROPEAN PATENT APPLICATION**

(43) Date of publication:
    **14.05.2003 Bulletin 2003/20**

(51) Int Cl.⁷: **B01D 39/16**

(21) Application number: **02024947.0**

(22) Date of filing: **06.11.2002**

(84) Designated Contracting States:
    **AT BE BG CH CY CZ DE DK EE ES FI FR GB GR
    IE IT LI LU MC NL PT SE SK TR**
    Designated Extension States:
    **AL LT LV MK RO SI**

(30) Priority: **09.11.2001 JP 2001344546
            06.06.2002 JP 2002166276**

(71) Applicant: **NITTO DENKO CORPORATION
    Osaka (JP)**

(72) Inventors:
    • **Maeoka, Takuya
      Ibaraki-shi, Osaka (JP)**
    • **Kawano, Eizo
      Ibaraki-shi, Osaka (JP)**

(74) Representative: **Grünecker, Kinkeldey,
    Stockmair & Schwanhäusser Anwaltssozietät
    Maximilianstrasse 58
    80538 München (DE)**

(54) **Antibacterial air filter medium and filter unit using the same**

(57)    An antibacterial air filter medium has at least one layer of a porous polytetrafluoroethylene film and at least one layer of an antibacterial reinforcing material which may be colored so as to make dirt inconspicuous; the antibacterial air filter medium retains its filtering performance even in an environment rich in water and can be inhibited from undergoing the growth of bacteria or mold fungi even in such an environment; and the antibacterial air filter medium, even when used after washing with water or in a high-humidity environment, does not undergo a decrease in filtering property such as particle collection efficiency and can be inhibited from undergoing the growth of bacteria or mold fungi thereon.

## FIG.1

EP 1 310 288 A1

**Description**

FIELD OF THE INVENTION

The present invention relates to an antibacterial air filter medium and a filter unit using the same.

BACKGROUND OF THE INVENTION

In facilities for the production of foods, medicines, or the like and in hospitals, highly clean surroundings from which microorganisms such as mold fungi and bacteria have been removed are required for securing product quality or from a hygienic standpoint. In general homes also, there is a growing interest in the removal of bacteria and mold fungi suspending in the living environment. Although bacteria and mold fungi can be caught with an air filter, the bacteria and the like caught may grow in the filter and come to fly off again or emit an offensive odor. It has hence been proposed to impart antibacterial properties to an air filter medium.

For example, Japanese Patent Laid-Open No. 42715/1987 discloses a filter medium obtained by laminating an antibacterial nonwoven fabric to an electret nonwoven fabric. Japanese Patent Laid-Open No. 42716/1987 discloses an electret nonwoven-fabric filter medium treated with an antibacterial agent. Furthermore, Japanese Patent Laid-Open No. 92113/1993 discloses a glass fiber filter medium having an antibacterial zeolite bonded with an acrylic binder.

However, the conventional filter media described above are unsuitable for use in a high-humidity environment or in an environment in which water is apt to splash on the filter. For example, in food production facilities where cleaning is periodically conducted, cleaning water splashes on the filter medium. As a result, there are cases where the fibers of the glass fiber filter medium hydrolyze or the electret filter undergoes a considerable decrease in performance. Since the environments which are rich in water usually have conditions under which microorganisms are apt to grow, an antibacterial filter medium is highly necessary which can retain the filtering performance even in such environments.

SUMMARY OF THE INVENTION

Accordingly, an aim of the invention is to provide an antibacterial filter medium capable of retaining its filtering performance even in environments rich in water. Another aim of the invention is to provide a filter unit using the filter medium.

For accomplishing those aims, the invention provides an antibacterial air filter medium which comprises at least one layer of a porous polytetrafluoroethylene (hereinafter referred to as "PTFE") film and at least one layer of an antibacterial reinforcing material. Since this antibacterial air filter medium is based on a combination of a porous PTFE film and an antibacterial reinforcing material, it can retain its filtering performance even when used in a high-humidity environment or even through washing with water. In this filter medium, at least one layer of reinforcing material has preferably been colored. The invention further provides a filter unit which comprises the antibacterial air filter medium.

BRIEF DESCRIPTION OF THE DRAWINGS

Fig. 1 is a sectional view illustrating one embodiment of the filter medium of the invention.
Fig. 2 is a sectional view illustrating another embodiment of the filter medium of the invention.
Fig. 3 is a slant view illustrating one embodiment of the filter unit of the invention.
Fig. 4 is a photograph showing the state of the reinforcing materials before airborne dust permeation in the Reference Examples.
Fig. 5 is a photograph showing the state of the reinforcing materials after airborne dust permeation in the Reference Examples.

<Description of the Reference Numerals>

1 porous PTFE film
2 antibacterial reinforcing material
3 air filter medium
4 supporting frame
5 spacer

**EP 1 310 288 A1**

DETAILED DESCRIPTION OF THE INVENTION

Preferred modes for carrying out the invention will be explained below.

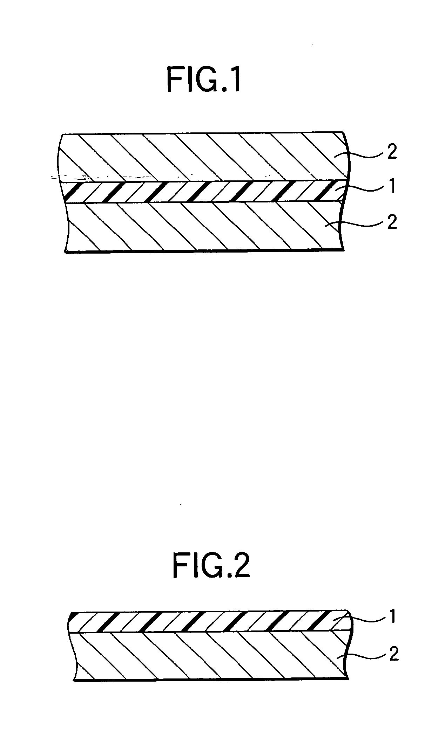

Fig. 1 and Fig. 2 are sectional views respectively illustrating embodiments of the filter medium of the invention. The embodiment shown in Fig. 1 comprises a porous PTFE film 1 and a pair of antibacterial reinforcing materials 2 respectively disposed on the upper and lower sides of the film 1. The embodiment shown in Fig. 2 comprises a porous PTFE film and an antibacterial reinforcing material 2 disposed on one side of the film. In the embodiment shown in Fig. 1, it is preferred that both of the reinforcing materials 2 be antibacterial. However, only either of the reinforcing materials may be antibacterial.

At least one layer of the reinforcing material 2 has preferably been colored. This coloration is intended to make the filter medium discoloration caused by collected dust particles inconspicuous. In particular, there are cases where in air filters used for general air conditioning, the discoloration caused by accumulated dirt attributable to airborne dust gives an unpleasant feeling to the user. However, when the filter medium has been colored beforehand, the discoloration is inconspicuous. Although both of the reinforcing materials in the embodiment shown in Fig. 1 are preferably colored, only either of these reinforcing materials may be colored. Furthermore, the filter medium shown in Fig. 1 may have a constitution in which one of the reinforcing materials is antibacterial and the other has been colored, or may have a constitution in which one or both of the reinforcing materials are antibacterial and have been colored.

Suitable as the antibacterial reinforcing material is an air-permeable member constituted of fibers having an antibacterial agent on the surface or in inner parts thereof. The form of the reinforcing material is not particularly limited as long as the material is more air-permeable than the porous PTFE film. The reinforcing material may be in the form of a nonwoven fabric, woven fabric, net, or the like. As the fibers may be used, for example, semisynthetic fibers such as cellulose or viscose rayon fibers or synthetic fibers such as polyester, polyolefin, polyamide, acrylic, polysulfone, poly(amide-imide), polyimide, poly(phenylene sulfide), or poly(vinylidene fluoride) fibers.

The antibacterial agent also is not particularly limited, and inorganic, organic, and natural antibacterial agents can be used alone or in combination of two or more thereof according to the intended use of the filter medium. However, preferred inorganic antibacterial agents are ones containing silver ions, copper ions, or zinc ions, and preferred organic antibacterial agents are ones having quaternary ammonium ions. Preferred natural antibacterial agents are ones comprising chitosan.

Examples of methods which can be used for fixing an antibacterial agent to fibers to be used as a reinforcing material include the impregnation method, adhesion method, and spraying method. Any of such methods may be used to adhere an antibacterial agent to the surface of fibers, to incorporate an antibacterial agent through kneading during fiber production, or to incorporate antibacterial ions into fibers by ion exchange.

Methods for coloration are not particularly limited. For example, a colorant such as a pigment or dye may be incorporated into the reinforcing material. From the standpoint of making the dirt inconspicuous, the reinforcing material is preferably colored in a gray tint.

The porous PTFE film 1 itself has water repellency. There is hence no particular need of taking care in using the filter medium in an environment rich in water, and any porous PTFE film can be used without particular limitations as long as it can exhibit the performance of an air filter. The porous PTFE film preferably has a thickness of from 5 to 200 μm, average pore diameter of from 0.1 to 30 μm, porosity of from 70 to 95%, and pressure loss of from 50 to 1,000 Pa (as measured at a permeation rate of 5.3 cm/sec by the method which will be described later).

Examples of techniques for uniting a porous PTFE film with a reinforcing material include a method in which a powder or web having a lower melting point than the constituent fibers of the reinforcing material and the PTFE is interposed between the porous PTFE film and the reinforcing material and this assemblage is heated. In the case where the constituted fibers of the reinforcing material have a lower melting point than the PTFE, the fibers of the reinforcing material may be melted to unite it with the porous PTFE film. Furthermore, use may be made of a method which comprises using an adhesive to unite the porous PTFE film with the reinforcing material. In this case, examples of the adhesive include two-pack type adhesives and adhesives of the thermal self-crosslinking type. Preferred two-pack type adhesives include epoxy resins, and preferred adhesives of the thermal self-crosslinking type include vinyl acetate/ethylene copolymers and ethylene/vinyl chloride copolymers.

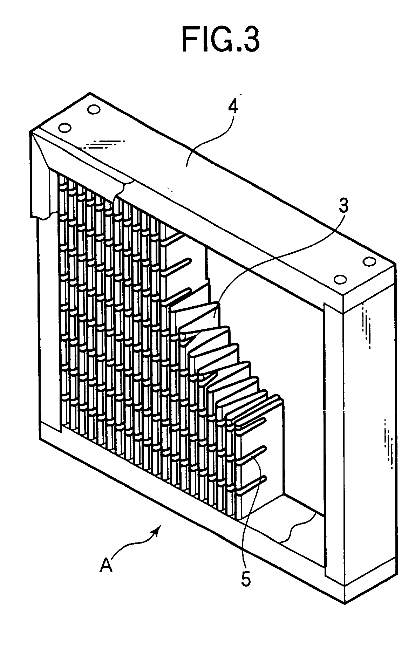

Fig. 3 is a slant view illustrating one embodiment of the filter unit of the invention. This filter unit comprises an air filter medium 3 comprising a porous PTFE film and an antibacterial reinforcing material. This filter medium 3 has been pleated into the shape of consecutive W's and housed in a supporting frame 4 (e.g., a frame made of a material having strength, such as a metal, plastic, fiber-reinforced plastic, or composite of two or more of them).

In this filter unit, the filter medium 3 has spacers 5 arranged in stripes on the surface thereof. These spacers 5 prevent the pleats of the filter medium 3, which has been pleated into the shape of W's, from coming into close contact with one another and thereby inhibit the pressure loss from increasing. Although various materials including plastics and metals can be used as the spacers, it is preferred to employ a porous material which does not impair the air permeability of the filter medium. It is also possible to dispose a nonwoven fabric, woven fabric, plastic film, or the like as a spacer between the pleats. Air cleaning with this filter unit can be accomplished by sending the air from the direction indicated by the arrow A in the figure.

As described above, according to the invention, an air filter medium and a filter unit using the same can be provide which, even when used after water washing or in a high-humidity environment, suffer no decrease in filtering property such as particle collection efficiency and can be inhibited from undergoing the growth of bacteria or mold fungi thereon.

The invention will be explained below in more detail by reference to Examples, but the invention should not be construed as being limited to the following Examples. Pressure loss, collection efficiency, and antifungal properties were determined by the following methods.

(Pressure Loss)

A sample was set on a circular holder having an effective area of 100 cm$^2$. A pressure difference was applied between the upstream side and the downstream side, and the air permeation rate was regulated to 5.3 cm/sec with a flowmeter. Under these conditions, the pressure loss was measured with a pressure gauge (manometer). With respect to each sample, ten areas were thus examined and the average of the found values was taken as the pressure loss of the sample.

(Collection Efficiency)

The same apparatus as in the measurement of pressure loss was used, and the air permeation rate was regulated to 5.3 cm/sec. Polydisperse dioctyl phthalate (DOP) was supplied to the upstream side so that the number of particles having a particle diameter of from 0.1 to 0.2 $\mu$m was about 10$^8$ per liter. The particle concentration on the upstream side and the concentration of particles which had passed through the sample to the downstream side were measured with a particle counter. The collection efficiency was determined using the following equation.

Collection efficiency (%) = [1 - (downstream-side particle concentration)/(upstream-side particle concentration)] x 100

In this measurement, the particles to be counted were limited to those having a particle diameter of from 0.1 to 0.2 $\mu$m.

(Antifungal Property)

Antifungal properties were examined in accordance with JIS Z 2911. A spore suspension prepared beforehand (wet method; mixture of four kinds) was dropped onto a sheet-form specimen and incubated for 7 days. Thereafter, the specimen was examined for mold growth. The state in which the area of the mold which had grown was not larger than 1/4 the whole dropping area was judged "antifungal", while the state in which that mold area was more than 1/4 the whole dropping area was judged "not antifungal".

EXAMPLE 1

A hundred parts by weight of a polytetrafluoroethylene powder (Fluon CD-123, manufactured by Asahi Glass Co., Ltd.) was evenly mixed with 30 parts by weight of a liquid lubricant (liquid paraffin). This mixture was preliminarily molded at 20 kg/cm$^2$ and then formed into a rod by extrusion molding. This red molding was passed through a pair of metallic pressure rolls to obtain a continuous sheet molding having a thickness of 0.2 mm. The liquid lubricant was removed from this sheet molding by extraction with n-decane. Thereafter, the sheet molding was wound on a tubular core.

This sheet molding was stretched in the lengthwise direction at 250°C and a stretch ratio of 20 by the roll stretching method and further stretched in the width direction at 100°C and a stretch ratio of 5 with a tenter. Thus, an unburned porous PTFE film (thickness, 15 $\mu$m; average pore diameter, 0.7 $\mu$m; porosity, 93%; pressure loss, 180 Pa) was obtained. This film was burned by heating at 380°C for 30 seconds.

An antibacterial nonwoven fabric having a basis weight of 12 g/m$^2$ (T0123WGM, manufactured by Unichika, Ltd.) was prepared as a reinforcing material to be laminated to the porous PTFE film obtained above. This antibacterial nonwoven fabric was disposed on each side of the porous PTFE film and these materials were laminated together by passing the resultant assemblage through 175°C rolls. The porous-PTFE air filter medium thus obtained was examined for pressure loss and collection efficiency. This filter medium was further subjected to an antifungal test by the method shown above. The results obtained are shown in Table 1. Furthermore, this porous-PTFE air filter medium was allowed to stand for 7 days in an environment having a temperature of 80°C and a humidity of 90%, and then examined for pressure loss and collection efficiency. The results obtained are shown in Table 2.

EXAMPLE 2

An antibacterial nonwoven fabric having a basis weight of 30 g/m$^2$ (Chemitac $\alpha$, manufactured by Teijin Ltd.) was prepared as a reinforcing material to be laminated to a porous PTFE film produced in the same manner as in Example 1. A heat-crosslinkable adhesive (S-900, manufactured by Sumitomo Chemical Co., Ltd.) was applied to one side of this antibacterial nonwoven fabric in an amount of 30 g/m$^2$. This nonwoven fabric was superposed on one side of the porous PTFE film and laminated thereto with a 175°C roll. The porous-PTFE air filter medium thus obtained was subjected to the same property evaluations as in Example 1. The results obtained are shown in Tables 1 and 2.

COMPARATIVE EXAMPLE 1

A nonwoven fabric of core/sheath structure having a basis weight of 30 g/m$^2$ (ELEVES T0303WDO, manufactured by Unichika, Ltd.) was prepared as a reinforcing material to be laminated to a porous PTFE film produced in the same manner as in Example 1. This nonwoven fabric had no antibacterial agent. This nonwoven fabric was disposed on each side of the porous PTFE film and these materials were laminated together by passing the resultant assemblage through 175°C rolls. The porous-PTFE air filter medium thus obtained was subjected to the same property evaluations as in Example 1. The results obtained are shown in Tables 1 and 2.

COMPARATIVE EXAMPLE 2

An antibacterial nonwoven fabric having a basis weight of 12 g/m$^2$ (T0123WGM, manufactured by Unichika, Ltd.) was thermally laminated to one side of a filter medium which was an electret of a nonwoven polypropylene fabric. This laminating was conducted by passing an assemblage of these materials through 140°C hot rolls. The air filter medium thus obtained was subjected to the same property evaluations as in Example 1. The results obtained are shown in Tables 1 and 2.

Table 1

|  | Pressure loss (Pa) | Collection efficiency (%) | Antifungal property |
|---|---|---|---|
| Example 1 | 220 | 99.999 | antifungal |
| Example 2 | 250 | 99.999 | antifungal |
| Comparative Example 1 | 200 | 99.999 | not antifungal |
| Comparative Example 2 | 300 | 99.995 | antifungal |

Table 2

| Properties after 7 days at 80°C, 90% RH | | |
|---|---|---|
|  | Pressure loss (Pa) | Collection efficiency (%) |
| Example 1 | 220 (±0) | 99.999 (±0) |
| Example 2 | 250 (±0) | 99.999 (±0) |
| Comparative Example 1 | 200 (±0) | 99.999 (±0) |
| Comparative Example 2 | 300 (±0) | 99.923 (-0.072) |
| * The value in each parenthesis indicates a change from the initial value. | | |

The filter media produced in Examples 1 and 2 were ascertained to be antifungal and to undergo no property deterioration even through standing in a high-temperature high-humidity environment. In contrast, the filter medium of Comparative Example 2 suffered a decrease in collection efficiency through standing in the high-temperature high-humidity environment.

REFERENCE EXAMPLES 1 AND 2

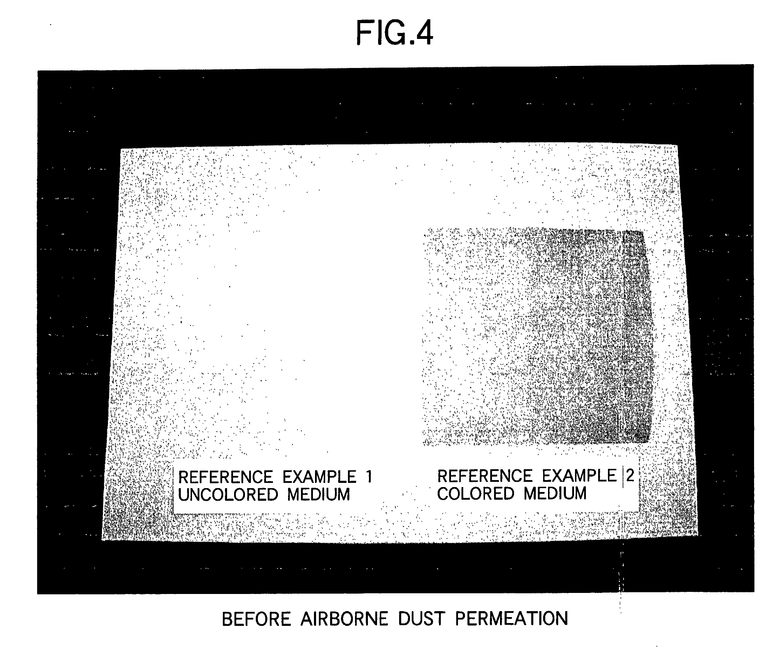

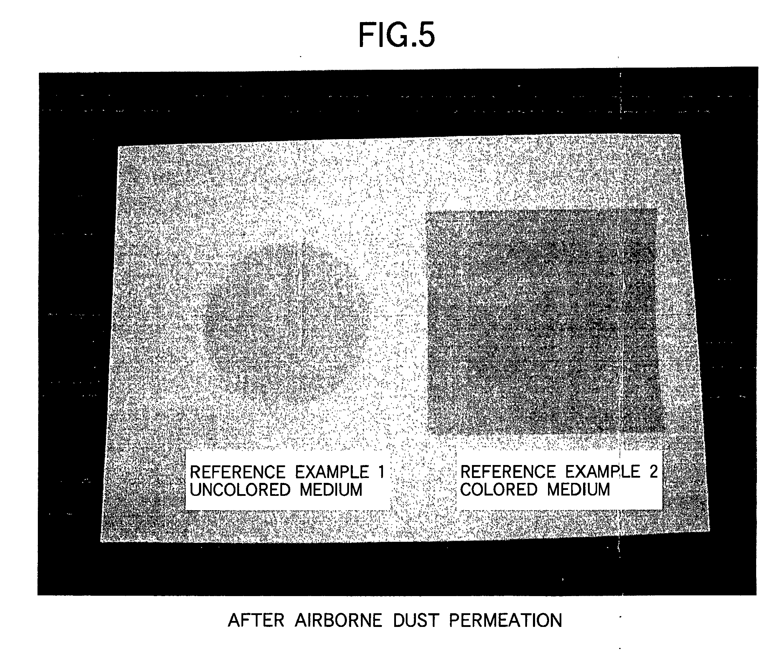

A reinforcing material which had been colored and a reinforcing material which had not been colored were examined for the discoloration caused by dust accumulation. The colored reinforcing material was a nonwoven fabric (SB46-50503GSO, manufactured by Unichika, Ltd.) formed from polyester fibers colored gray by incorporating carbon therein. The reinforcing material which had not been colored was the nonwoven fabric used in Comparative Example 1. Airborne dust was caused to permeate through these reinforcing materials at a flow rate of 5.3 cm/sec for 14 days, and the reinforcing materials were then compared in the degree of dirt. The results obtained are shown in Figs. 4 and 5.

Furthermore, an air filter medium was produced in the same manner as in Example 1, except that use was made of an antibacterial colored nonwoven fabric obtained by incorporating carbon into the antibacterial nonwoven fabric used in Example 1 to color the fabric gray. This filter medium showed the same properties as that obtained in Example 1.

While the invention has been described in detail and with reference to specific embodiments thereof, it will be apparent to one skilled in the art that various changes and modifications can be made therein without departing from the spirit and scope thereof.

**Claims**

1. An antibacterial air filter medium which comprises at least one layer of a porous polytetrafluoroethylene film and at least one layer of an antibacterial reinforcing material.

2. The antibacterial air filter medium as claimed in claim 1, wherein at least one layer of said reinforcing material is colored.

3. A filter unit which comprises the antibacterial air filter medium comprising at least one layer of a porous polytetrafluoroethylene film and at least one layer of an antibacterial reinforcing material.

4. The filter unit as claimed in claim 3, wherein at least one layer of said reinforcing material is colored.

5. The antibacterial air filter medium as claimed in claim 1, wherein said reinforcing material is in the form of a nonwoven fabric, a woven fabric or a net.

6. The antibacterial air filter medium as claimed in claim 1, wherein said reinforcing material is made of cellulose fibers, viscose rayon fibers, polyester fibers, polyolefin fibers, polyamide fibers, acrylic fibers, polysulfone fibers, poly(amide-imide) fibers, polyimide fibers, poly(phenylene sulfide) fibers or poly(vinylidene fluoride) fibers.

7. The antibacterial air filter medium as claimed in claim 1, wherein said reinforcing material has inorganic antibacterial agents, organic antibacterial agents, natural antibacterial agents or a mixture thereof.

8. The antibacterial air filter medium as claimed in claim 1, wherein said porous polytetrafluoroethylene film has a thickness of from 5 to 200 $\mu$m, average pore diameter of from 0.1 to 30 $\mu$m, porosity of from 70 to 95% and pressure loss of from 50 to 1,000 Pa as measured at a permeation rate of 5.3 cm/sec.

9. The filter unit as claimed in claim 3, wherein said reinforcing material is in the form of a nonwoven fabric, a woven fabric or a net.

10. The filter unit as claimed in claim 3, wherein said reinforcing material is made of cellulose fibers, viscose rayon fibers, polyester fibers, polyolefin fibers, polyamide fibers, acrylic fibers, polysulfone fibers, poly(amide-imide) fibers, polyimide fibers, poly(phenylene sulfide) fibers or poly(vinylidene fluoride) fibers.

11. The filter unit as claimed in claim 3, wherein said reinforcing material has inorganic antibacterial agents, organic antibacterial agents, natural antibacterial agents or a mixture thereof.

12. The filter unit as claimed in claim 3, wherein said porous polytetrafluoroethylene film has a thickness of from 5 to 200 $\mu$m, average pore diameter of from 0.1 to 30 $\mu$m, porosity of from 70 to 95% and pressure loss of from 50 to 1,000 Pa as measured at a permeation rate of 5.3 cm/sec.

## FIG.1

## FIG.2

# FIG.3

# FIG.4

REFERENCE EXAMPLE 1
UNCOLORED MEDIUM

REFERENCE EXAMPLE 2
COLORED MEDIUM

BEFORE AIRBORNE DUST PERMEATION

EP 1 310 288 A1

# FIG.5

REFERENCE EXAMPLE 1
UNCOLORED MEDIUM

REFERENCE EXAMPLE 2
COLORED MEDIUM

AFTER AIRBORNE DUST PERMEATION

**European Patent**
**Office**

## EUROPEAN SEARCH REPORT

Application Number

EP 02 02 4947

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | PATENT ABSTRACTS OF JAPAN vol. 18, no. 30, 17 January 1994 (1994-01-17) & JP 05 261145 A (JAPAN GORE TEX), 12 October 1993 (1993-10-12) * abstract * | 1,3,5 | B01D39/16 |
| A | DATABASE WPI Week 198627 Derwent Publications Ltd., London, GB; AN 1986-171597 XP002234393 & JP 61 102469 A (KANAI), 21 May 1986 (1986-05-21) * abstract * | 1,3,5, 8-12 | |
| A | EP 0 960 644 A (DAIKIN INDUSTRIES) 1 December 1999 (1999-12-01) * claims 1-5,9 * | 1 | |
| A | WO 94 04211 A (FLOW-METER) 3 March 1994 (1994-03-03) * page 5, line 6 - line 27; claims 1,4,5,10 * | 1 | TECHNICAL FIELDS SEARCHED (Int.Cl.7) B01D |

The present search report has been drawn up for all claims

| Place of search | Date of completion of the search | Examiner |
|---|---|---|
| BERLIN | 12 March 2003 | Bertram, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 02 02 4947

This annex lists the patent family members relating to the patent documents cited in the above-mentioned European search report.
The members are as contained in the European Patent Office EDP file on
The European Patent Office is in no way liable for these particulars which are merely given for the purpose of information.

12-03-2003

| Patent document cited in search report | | Publication date | Patent family member(s) | | Publication date |
|---|---|---|---|---|---|
| JP 05261145 | A | 12-10-1993 | NONE | | |
| JP 61102469 | A | 21-05-1986 | NONE | | |
| EP 960644 | A | 01-12-1999 | EP | 0960644 A1 | 01-12-1999 |
| | | | CN | 1244134 T | 09-02-2000 |
| | | | WO | 9831451 A1 | 23-07-1998 |
| | | | TW | 406101 B | 21-09-2000 |
| WO 9404211 | A | 03-03-1994 | WO | 9404211 A1 | 03-03-1994 |
| | | | DE | 69230295 D1 | 16-12-1999 |
| | | | DE | 69230295 T2 | 23-03-2000 |
| | | | EP | 0659096 A1 | 28-06-1995 |

EPO FORM P0459

For more details about this annex : see Official Journal of the European Patent Office, No. 12/82